Europäisches Patentamt
European Patent Office
Office européen des brevets

(19)

(11) Publication number: **0 202 762 A1**

(12) **EUROPEAN PATENT APPLICATION**

(21) Application number: 86302805.6

(22) Date of filing: 15.04.86

(51) Int. Cl.[4]: C07D 417/12 , A01N 47/36

(30) Priority: 16.04.85 US 723655

(43) Date of publication of application: 26.11.86 Bulletin 86/48

(84) Designated Contracting States: AT BE CH DE FR GB IT LI LU NL SE

(71) Applicant: E.I. DU PONT DE NEMOURS AND COMPANY
1007 Market Street
Wilmington Delaware 19898(US)

(72) Inventor: Hanagan, Mary Ann
17 Barclay Court
Blue Bell Pennsylvania 19422(US)
Inventor: Pasteris, Robert James
305 Plymouth Road
Wilmington Delaware 19898(US)

(74) Representative: Hildyard, Edward Martin et al
Frank B. Dehn & Co. Imperial House 15-19 Kingsway
London WC2B 6UZ(GB)

# (54) Herbicidal sulfonamide.

(57) 2-Butyl-N-[4,6-dimethoxypyrimidin-2-yl)-aminocarbonyl]-2,3-dihydro-5-methoxy-1,2-benzisothiazole-7-sulfonamide, S,S-dioxide, (Formula I), and its agriculturally suitable salts, shows a selective preemergent and postemergent activity, especially on sugar beet.

The novel compound may be made e.g. by reacting 2-butyl-2,3-dihydro-5-methoxy-1,2-benzisothiazole-7-sulfonamide-1,1-dioxide with phenyl-(4,6-dimethoxy-2-pyrimidin-2-yl-carbamate.

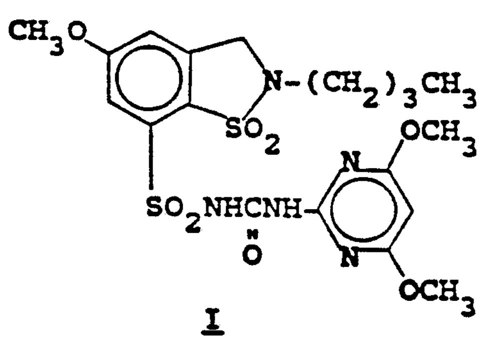

I

EP 0 202 762 A1

# HERBICIDAL SULFONAMIDE

## Background of the Invention

This invention relates to a specific sulfonamide, its agriculturally suitable compositions, and its method-of-use as a selective pre-emergent and/or post-emergent herbicide.

The compound of this invention is generically disclosed in European Patent Application EP-A-107,979 which was published May 9, 1984, disclosing herbicidal sulfonylureas of the formula:

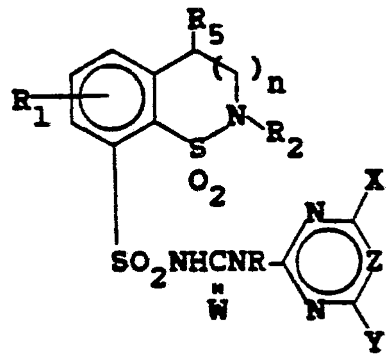

wherein

$R_1$ is H, F, Cl, Br, $CH_3$, $OCH_3$, $CF_3$, $SCH_3$, or $OCF_2H$;

$R_2$ is H or $C_1$-$C_4$ alkyl;

n is 0 or 1;

W is O or S;

R is H or $CH_3$;

$R_5$ is H or $CH_3$;

Z is CH, N, $CCH_3$, $CC_2H_5$, CCl or CBr;

X is $CH_3$, $OCH_3$, $OC_2H_5$, Cl, F, Br, $OCF_2H$, $CH_2F$ or $CF_3$; and

Y is, inter alia, H, $CH_3$, $OCH_3$, $OC_2H_5$, $CH_2OCH_3$, $NH_2$, etc.

Compound I is also generically disclosed in South African Patent Application 83/5165, published 1/16/84, which discloses herbicidal sulfonamides of formula

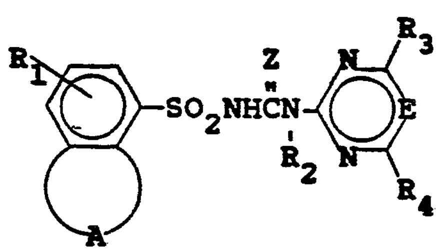

wherein

A is an unsubstituted or substituted bridge of 3 or 4 atoms which contains 1 or 2 oxygen, sulfur or nitrogen atoms and, together with the linking carbon atom, forms a non-aromatic 5-or 6-membered heterocyclic ring system, with the proviso that two oxygen atoms are separated by at least one carbon atom and that oxygen and sulfur atoms are only linked to each other if the sulfur atom takes the form of the -SO-or $SO_2$-group, and $R_2$ is hydrogen, halogen, nitro, $C_1$-$C_4$ alkyl, $C_1$-$C_4$ haloalkyl, $C_1$-$C_4$ alkoxy, $C_1$-$C_2$ haloalkoxy, $C_1$-$C_4$ alkoxycarbonyl, $C_1$-$C_4$ alkylthio, $C_1$-$C_4$ alkylsulfinyl, $C_1$-$C_4$ alkylsulfonyl or $C_2$-$C_5$ alkoxyalkoxy;

$R_1$ is H, halogen, $NO_2$, $C_1$-$C_4$ alkyl, $C_1$-$C_4$ haloalkyl, $C_1$-$C_4$ alkoxy, $C_1$-$C_4$ haloalkoxy, $C_1$-$C_4$ alkoxycarbonyl, $C_1$-$C_4$ alkylthio, $C_1$-$C_4$ alkylsulfinyl, $C_1$-$C_4$ alkylsulfonyl or $C_2$-$C_5$ alkoxyalkoxy;

$R_3$ and $R_4$, each independently of the other, are hydrogen, $C_1$-$C_4$ alkyl, $C_1$-$C_4$ alkoxy, $C_1$-$C_4$ haloalkoxy, $C_1$-$C_4$ haloalkylthio, $C_1$-$C_4$ alkylthio, halogen, $C_2$-$C_5$ alkoxyalkoxy or $NR_5R_6$, wherein $R_5$ and $R_6$ are hydrogen or $C_1$-$C_4$ alkyl; and

E is CH or N.

Despite the broad teachings of these two disclosures, there is still a need for specific compounds which demonstrate selectivity as pre-emergent and/or post-emergent herbicides.

Summary of the Invention

According to the instant invention such a compound has unexpectedly been found. The invention relates to the compound of Formula I, its agriculturally suitable compositions, and its method-of-use as a selective pre-emergent and/or post-emergent herbicide, particularly for use in sugar beets.

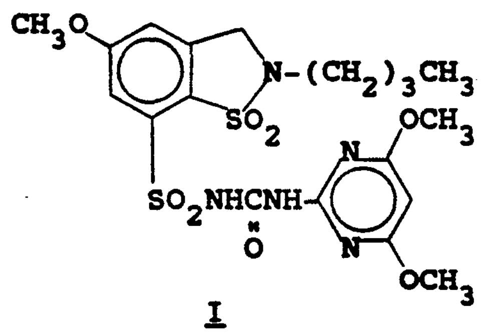

I

This compound is 2-butyl-N-[4,6-dimethoxypyrimidin-2-yl)-aminocarbonyl]-2,3-dihydro-5-methoxy-1,2-benzisothiazole-7-sulfonamide, S,S-dioxide, m.p. 174-176°C.

Detailed Description of the Invention

Three processes for preparing the compound of Formula I are described below; preferred reaction conditions are given by way of illustration.

The compound of Formula I can be prepared by the method described in Equation 1.

Equation 1

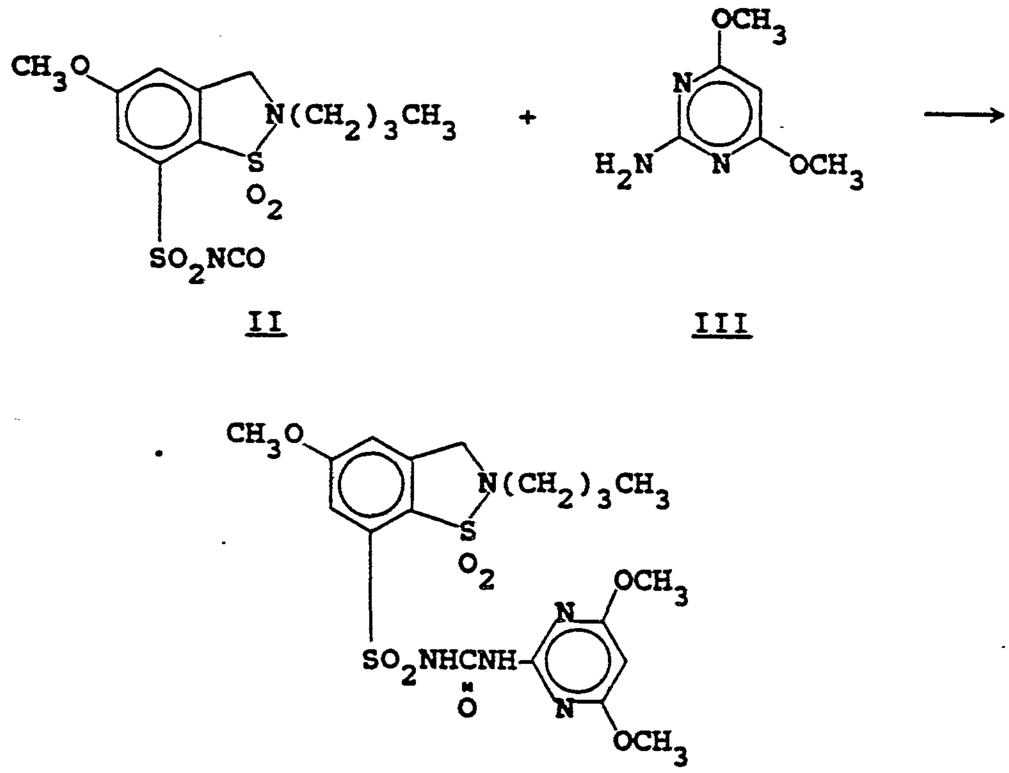

II

III

I

The sulfonyl isocyanate of Formula II is reacted with the aminopyrimidine of Formula III in an inert solvent such as dichloromethane, acetonitrile, xylene or chlorobenzene, for a period of 1 to 96 hours at temperatures of 20° to 100°C. The product of Equation 1 can be isolated either by filtration or by evaporation of the reaction solvent and trituration with a solvent such as 1-chlorobutane, ether or similar solvents.

The sulfonyl isocyanate of Formula II can be prepared by methods, or modifications thereof, obvious to one skilled in the art as described in U.S. Patent 4,379,769.

The compound of Formula I can also be prepared by the method shown in Equation 2 by reacting the sulfonamide of Formula IV with the phenylcarbamate of Formula V in the presence of a molar equivalent of 1,8-diazabicyclo[5.4.0]undec-7-ene. The phenylcarbamate of Formula V can be prepared by the reaction of the aminopyrimidine III with diphenyl carbonate in the presence of a base such as sodium hydride or, alternatively, by the reaction of the aminopyrimidine of Formula III with phenyl chloroformate in the presence of an acid acceptor such as pyridine or triethylamine.

Equation 2

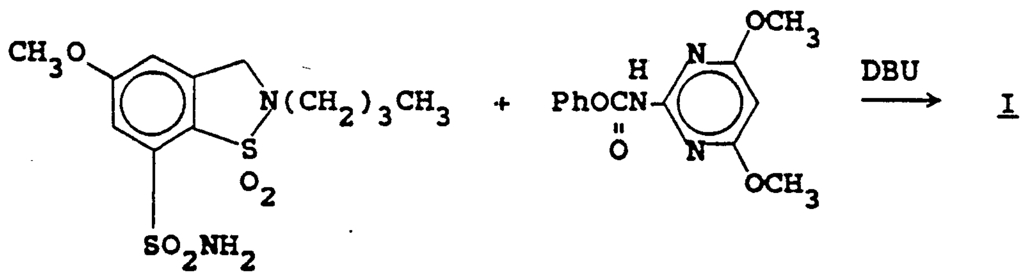


IV V

Also, the compound of Formula I can be prepared, as shown in Equation 3, by reacting the aminopyrimidine of Formula III with the phenylcarbamate of Formula VI at temperatures of 25° to 100°C in solvents such as dioxane, acetonitrile or tetrahydrofuran for a period of (about) 1 to 24 hours. The phenylcarbamate of Formula VI can be prepared by reaction of the sulfonamide IV with phenyl chloroformate in the presence of a base such as pyridine or sodium hydroxide.

Equation 3

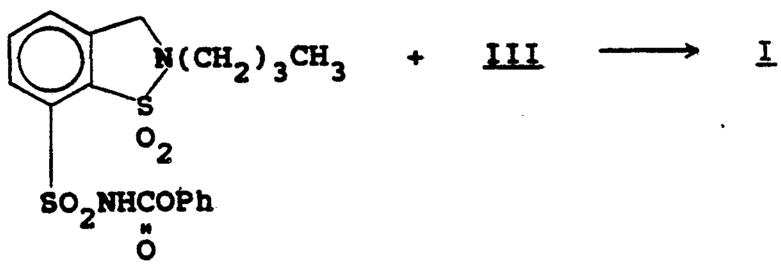

VI

The sulfonamide of Formula IV can be prepared starting with the t-butylsulfonamide of Formula VII as shown in Equation 4.

Equation 4

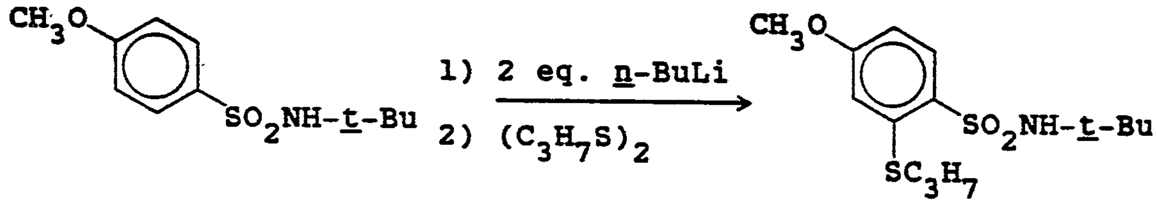


VII VIII

VIII

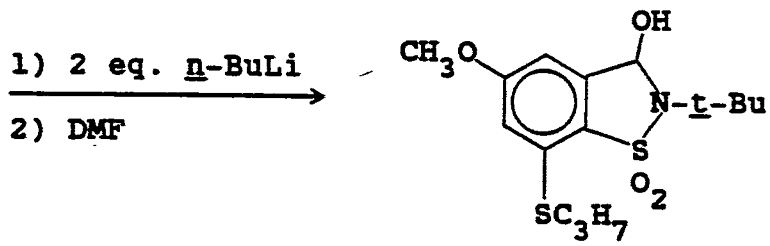


IX

IX

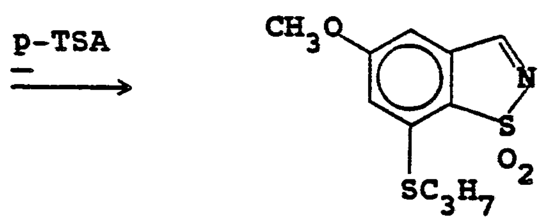


X

X

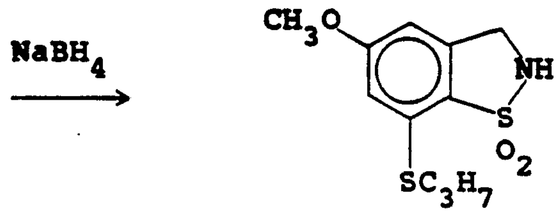


XI

XI

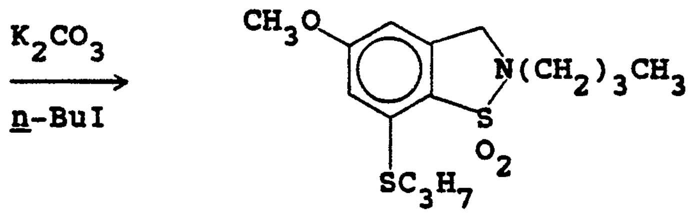


XII

XII

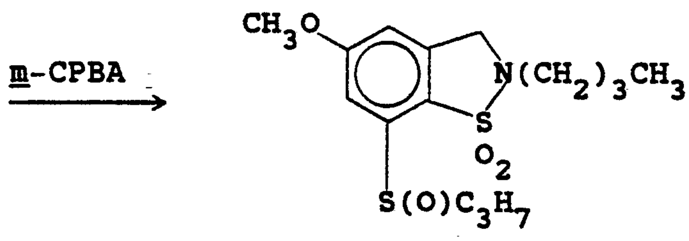


XIII

XIII

1) $Cl_2$

2) $NH_3$

IV

The aminopyrimidine of Formula III can be prepared by methods obvious to one skilled in the art. For a review of the syntheses and reactions of 2-aminopyrimidines, see The Chemistry of Heterocyclic Compounds, Vol. 16, John Wiley and Sons (1962).

Agriculturally suitable salts of the compound of Formula I are also useful herbicides and can be prepared in a number of ways known to the art. For example, metal salts can be made by contacting the compound of Formula I with a solution of an alkali or alkaline earth metal salt having a sufficiently basic anion (e.g., hydroxide, alkoxide or carbonate). Quaternary amine salts can be made by similar techniques.

Salts of the compound of Formula I can also be prepared by exchange of one cation for another. Cationic exchange can be effected by direct contact of an aqueous solution of a salt of the compound of Formula I (e.g., an alkali metal or quaternary amine salt) with a solution containing the cation to be exchanged. This method is most effective when the desired salt containing the exchanged cation is insoluble in water and can be separated by filtration.

Exchange may also be effected by passing an aqueous solution of a salt of the compound of Formula I (e.g. an alkali metal or quaternary amine salt) through a column packed with a cation exchange resin containing the cation to be exchanged for that of the original salt and the desired product is eluted from the column. This method is particularly useful when the desired salt is water-soluble.

Acid addition salts, useful in this invention, can be obtained by reacting the compound of Formula I with a suitable acid (e.g., p-toluenesulfonic acid, trichloroacetic acid or the like).

The preparation of the compound of this invention is further illustrated by the following examples wherein temperatures are given in degrees Celsius unless otherwise designated.

Example 1

2-Propylthio-4-methoxy-N-(1,1-dimethylethyl)-benzenesulfonamide

To 4-methoxy-N-(1,1-dimethylethyl)-benzenesulfonamide (100 g) stirring at -78° in tetrahydrofuran (1 L) under nitrogen was added dropwise 1.55 M n-butyllithium (0.584 L). The reaction mixture was allowed to warm to room temperature and stirred for 1 hour at that temperature. The solution was then re-cooled to -78° and dipropyldisulfide (0.0711 L) was added dropwise. The reaction mixture was allowed to warm to room temperature and stirred overnight. The whole mixture was poured into water and extracted with ether. The ethereal solution was washed successively with water and saturated sodium chloride solution, dried over sodium sulfate and concentrated in vacuo to yield 105.5 g of the title compound, m.p. 84-88°.

Example 2

2-(1,1-Dimethylethyl)-2,3-dihydro-5-methoxy-7-(propylthio)-1,2-benzisothiazol-3-ol-1,1-dioxide

A solution 2-propylthio-4-methoxy-N-(1,1-dimethylethyl)benzenesulfonamide (250.42 g) in tetrahydrofuran (1.5 L) was cooled to 0° and n-butyllithium was added cautiously with stirring of the solution under a nitrogen atmosphere. The reaction mixture was allowed to warm to room temperature and stirred for 1.5 hours. After this time, it was cooled to -78° and N,N-dimethylformamide - (0.184 L) was added dropwise. The mixture was allowed to warm to room temperature and stir overnight. The whole was quenched with water and the aqueous mixture extracted with ethyl acetate. The organic layer was washed with water and saturated sodium chloride solution and dried over sodium sulfate. The solvent(s) was removed by rotary evaporation and the crude oily product triturated with butyl chloride to give 149 g of the title compound as a yellow solid, m.p. 99-102°.

Example 3

5-Methoxy-7-(propylthio)-1,2-benzisothiazole-1,1-dioxide

Benzene (0.40 L) containing 2-(1,1-dimethylethyl)-2,3-dihydro-5-methoxy-7-(propylthio)-1,2-benzisothiazol-3-ol-1,1-dioxide - (147.2 g) and p-toluenesulfonic acid (1.5 g) was azeotroped for 5 hours. After this time, 7.9 mL of water had been collected. The reaction mixture was allowed to cool and the crude product was collected by filtration to yield 63.5 g of the title compound as a yellow solid, m.p. 109-113°.

Example 4

2,3-Dihydro-5-methoxy-7-(propylthio)-1,2-benzisothiazole-1,1-dioxide

5-methoxy-7-(propylthio)-1,2-benzisothiazole-1,1-dioxide (83 g) was suspended in ethanol/tetrahydrofuran (1:0.4 L) and cooled to 0°. Sodium borohydride (11.60 g) was added in portions to the cool stirred suspension and the resulting solution was allowed to warm to room temperature and stirred overnight. Acetic acid (0.06 L) was added and a white precipitate formed. The precipitate was collected by filtration, washed with water and dried to yield 68 g of the title compound as a white solid, m.p. 85-89°.

Example 5

2-Butyl-2,3-dihydro-5-methoxy-7-(propylthio)-1,2-benzisothiazole-1,1-dioxide

Powdered potassium carbonate (15.2 g) was suspended in ethanol (0.20 L) and 2,3-dihydro-5-methoxy-7-(propylthio)-1,2-benzisothiazole-1,1-dioxide (20.0 g) and 1-iodobutane (excess) were added. The reaction mixture was refluxed overnight and its course was monitored by TLC. After this time, the whole mixture was poured into water, acidified with 10% hydrochloric acid solution and extracted with 1-chlorobutane. The organic layer was washed with water, dried over sodium sulfate and concentrated in vacuo to yield 20 g of the subject compound as a yellow oil.

Example 6

2-Butyl-2,3-dihydro-5-methoxy-7-(propylsulfinyl)-1,2-benzisothiazole-1,1-dioxide

To 2-butyl-2,3-dihydro-5-methoxy-7-(propylsulfinyl)-1,2-benzisothiazole-1,1-dioxide (20.0 g) in methylene chloride (0.20 L) at 0° was added m-chloroperoxybenzoic acid (13.0 g) in methylene chloride (0.10 L). The reaction mixture was stirred at 0° for 0.5 hours and saturated sodium sulfite (0.010 L) was added. The whole mixture was poured into water and extracted with methylene chloride. The organic layer was washed 2x with saturated sodium bicarbonate solution, water and saturated sodium chloride solution. The extract was dried over sodium sulfate, concentrated in vacuo and triturated with hexane/1-chlorobutane mixture to yield 14.0 g of the title compound as a semisolid.

Example 7

2-Butyl-2,3-dihydro-5-methoxy-1,2-benzisothiazole-7-sulfonamide-1,1-dioxide

2-Butyl-2,3-dihydro-5-methoxy-7-(propylsulfinyl)-1,2-benzisothiazole-1,1-dioxide (19.0 g) was dissolved in propionic acid and cooled to -20°. Water (1.5 mL) and then liquid chlorine -(4.4 mL) are added. The reaction mixture was stirred at -20° for 0.5 hours then allowed to warm to room temperature. The reaction mixture was poured into ice and water and extracted with ethyl acetate. The extracts were washed 3 times with saturated sodium bicarbonate, then with saturated sodium chloride solution, dried over sodium sulfate and concentrated in vacuo to yield the sulfonyl chloride as a pale yellow oil. The whole was dissolved in methylene chloride, cooled to -40° and liquid ammonia (3.4 mL) was added dropwise. The reaction mixture was allowed to warm gradually to room temperature, after which the mixture was poured into water, acidified with 10% hydrochloric acid solution and extracted with ethyl acetate. The organic layer was washed with water and saturated sodium chloride solution, dried over sodium sulfate and concentrated by rotary evaporation to yield 9.26 of the title compound as a white solid, m.p. 164-166°.

Example 8

1,2-Benzisothiazole-7-sulfonamide, 2-butyl-N(((4,6-dimethoxypyrimidin-2-yl)aminocarbonyl))-2,3-dihydro-5-methoxy-1,2-dioxide

To a stirred solution of 2-butyl-2,3-dihydro-5-methoxy-1,2-benzisothiazole-7-sulfonamide-1,1-dioxide (0.5 g) and 1,8-diazabicyclo[5.4.0]undec-7-ene (0.22 mL) in acetonitrile (3.0 mL) was added phenyl-(4,6-dimethoxy-2-pyrimidin-2-yl)carbamate -(0.41 g). The reaction mixture was stirred at room temperature for 1 hour. After this time, water (6.0 mL) and 10% hydrochloric acid solution were added. The resulting precipitate was collected by filtration, washed 3 times with water and vacuum dried to yield 0.68 g of the subject compound as a white solid, m.p. 174-176°.

Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following approximate proportions:

Table 1

| | Active Ingredient | Weight Percent* Diluent(s) | Surfactant(s) |
|---|---|---|---|
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a surfactant or a diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, December 4, 1967, pp. 147ff. and "Perry's Chemical Engineer's Handbook", 5th Ed., McGraw-Hill, New York, 1973, pp. 8-57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through Col. 5, line 17 and Examples 1-4;

G. C. Klingman, "Weed Control as a Science", John Wiley and Sons, Inc., New York, 1961, pp.

81-96; and

J. D. Fryer and S. A. Evans, "Weed Control Handbook", 5th Ed., Blackwell Scientific Publications, Oxford, 1968, pp. 101-103.

In the following examples, all parts are by weight unless otherwise indicated.

Example 9

Wettable Powder

| | |
|---|---|
| 2-Butyl-N-[(4,6-dimethoxypyrimidin-2-yl)aminocarbonyl]-2,3-dihydro-5-methoxy-1,2-benzisothiazole-7-sulfonamide, S,S-dioxide | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, re-blended, and packaged.

Example 10

Wettable Powder

| | |
|---|---|
| 2-Butyl-N-[(4,6-dimethoxypyrimidin-2-yl)aminocarbonyl]-2,3-dihydro-5-methoxy-1,2-benzisothiazole-7-sulfonamide, S,S-dioxide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 11

Granule

| | |
|---|---|
| Wettable Powder of Example Above | 5% |
| attapulgite granules (U.S.S. 20-40 mesh; 0.84-0.42 mm) | 95% |

A slurry of wettable powder containing 25% solids is sprayed on the surface of attapulgite granules in a double-cone blender. The granules are dried and packaged.

Example 12

Extruded Pellet

| | |
|---|---|
| 2-Butyl-N-[(4,6-dimethoxypyrimidin-2-yl)aminocarbonyl]-2,3-dihydro-5-methoxy-1,2-benzisothiazole-7-sulfonamide, S,S-dioxide | 25% |
| anhydrous sodium sulfate | 10% |
| crude calcium ligninsulfonate | .5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 13

Low Strength Granule

| | |
|---|---|
| 2-Butyl-N-[(4,6-dimethoxypyrimidin-2-yl)aminocarbonyl]-2,3-dihydro-5-methoxy-1,2-benzisothiazole-7-sulfonamide, S,S-dioxide | 0.1% |
| attapulgite granules (U.S.S. 20-40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 14

Granule

| | |
|---|---|
| 2-Butyl-N-[(4,6-dimethoxypyrimidin-2-yl)aminocarbonyl]-2,3-dihydro-5-methoxy-1,2-benzisothiazole-7-sulfonamide, S,S-dioxide | 80% |
| wetting agent | 1% |
| crude ligninsulfonate salt (containing 5-20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14-100 mesh - (1410-1419 microns), and packaged for use.

Example 15

Low Strength Granule

| | |
|---|---|
| 2-Butyl-N-[(4,6-dimethoxypyrimidin-2-yl)aminocarbonyl]-2,3-dihydro-5-methoxy-1,2-benzisothiazole-7-sulfonamide, S,S-dioxide | 1% |
| N,N-dimethylformamide | 9% |
| attapulgite granules (U.S.S. 20-40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example 16

Aqueous Suspension

| | |
|---|---|
| 2-Butyl-N-[(4,6-dimethoxypyrimidin-2-yl)aminocarbonyl]-2,3-dihydro-5-methoxy-1,2-benzisothiazole-7-sulfonamide, S,S-dioxide | 40.0% |
| polyacrylic acid thickener | 0.3% |
| dodecylphenol polyethylene glycol ether | 0.5% |
| disodium phosphate | 1.0% |
| monosodium phosphate | 0.5% |
| polyvinyl alcohol | 1.0% |
| water | 56.7% |

The ingredients are blended and ground together in a sand mill to produce particles essentially all under 5 microns in size.

Example 17

Solution

| | |
|---|---|
| 2-Butyl-N-[(4,6-dimethoxypyrimidin-2-yl)aminocarbonyl]-2,3-dihydro-5-methoxy-1,2-benzisothiazole-7-sulfonamide, S,S-dioxide, sodium salt | 5% |
| water | 95% |

The salt is added directly to the water with stirring to produce the solution, which may then be packaged for use.

Example 18

High Strength Concentrate

| | |
|---|---|
| 2-Butyl-N-[(4,6-dimethoxypyrimidin-2-yl)aminocarbonyl]-2,3-dihydro-5-methoxy-1,2-benzisothiazole-7-sulfonamide, S,S-dioxide | 99.0% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 19

Wettable Powder

| | |
|---|---|
| 2-Butyl-N-[(4,6-dimethoxypyrimidin-2-yl)aminocarbonyl]-2,3-dihydro-5-methoxy-1,2-benzisothiazole-7-sulfonamide, S,S-dioxide | 90.0% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 20

Oil Suspension

| | |
|---|---|
| 2-Butyl-N-[(4,6-dimethoxypyrimidin-2-yl)aminocarbonyl]-2,3-dihydro-5-methoxy-1,2-benzisothiazole-7-sulfonamide, S,S-dioxide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

Example 21

Dust

| | |
|---|---|
| 2-Butyl-N-[(4,6-dimethoxypyrimidin-2-yl)aminocarbonyl]-2,3-dihydro-5-methoxy-1,2-benzisothiazole-7-sulfonamide, S,S-dioxide | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

Example 22

**Oil Suspension**

| | |
|---|---|
| 2-Butyl-N-[(4,6-dimethoxypyrimidin-2-yl)aminocarbonyl]-2,3-dihydro-5-methoxy-1,2-benzisothiazole-7-sulfonamide, S,S-dioxide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 23

**Wettable Powder**

| | |
|---|---|
| 2-Butyl-N-[(4,6-dimethoxypyrimidin-2-yl)aminocarbonyl]-2,3-dihydro-5-methoxy-1,2-benzisothiazole-7-sulfonamide, S,S-dioxide | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3 mm opening) and packaged.

UTILITY

The compound of this invention is particularly useful for the control of weeds in sugar beets and fodder beets. This is a crop that takes a long period to become established. In this interval, the crop seedlings must be nurtured carefully, with particular attention to weed control to prevent damage due to competition. The subject compound can be used either pre-or postemergence and will control numerous problem weeds including galium - (Galium aparine ), pigweed (Amaranthus spp.), lambsquarter (Chenopodium album), wild oats (Avena fatua), wild radish (Raphanus raphanistrum), and blackgrass (Alopecurus myosuroides).

The rate of application for this compound is determined by a number of factors including the weeds to be controlled, weather and climate, soil type, time of application, age and size of crop and weeds, method of application (pre or post), etc. In general terms, the rate will vary between about 25 and 250 g/ha. The rate to be used in any given situation can be selected by one with ordinary skill in the art.

This compound can and will often be used in mixtures with one or more other herbicides. It may be mixed with any other herbicide selective on beets including metamitron, phenmedipham, chloridazin, desmedipham, lenacil, ethofumesate, cycloate, diclofop-methyl, fuazifop, haloxyfop and Assure®.

The selective properties of this compound were discovered in greenhouse tests. The results of these tests are shown in the tables below.

Test A

Postemergence

Two round pans (25 cm diameter by 12.5 cm deep) were filled with Sassafras sandy loam soil. One pan was planted with blackgrass (Alopecurus myosuroides), sugar beets, nutsedge (Cyperus rotundus) tubers, rape (Brassica napus ), crabgrass (Digitaria sanguinalis), sicklepod (Cassia ob-

tusifolia), teaweed (Sida Spinosa), jimsonweed - (Datura stramonium), velvetleaf (Abutilon theophrasti ), and giant foxtail (Setaria faberii). The other pan was planted with wheat, cotton, rice, corn, soybean, wild oats ( Avena fatua), cocklebur - (Xanthium pensylvanicum), morningglory (Ipomoea hederacea), johnsongrass (Sorghum halepense ) and barnyardgrass (Echinochloa crusgalli). The plants were grown for approximately fourteen days, then sprayed postemergence with Compound I dissolved in a non-phytotoxic solvent.

Preemergence

Two round pans (25 cm diameter by 12.5cm deep) were filled with Sassafras sandy loam soil. One pan was planted with blackgrass, sugar beets, nutsedge, rape, crabgrass, sicklepod, teaweed, jimsonweed, velvetleaf and giant foxtail. The other pan was planted with wheat, cotton, rice, corn, soybeans, wild oats, cocklebur, morningglory, johnsongrass and barnyardgrass. The two pans were sprayed preemergence with Compound I dissolved in a non-phytotoxic solvent.

Treated plants and controls were maintained in the greenhouse for 28 days, then all treated plants were compared to controls and visually rated for plant response.

Response ratings are based on a scale of 0 to 100 where 0 = no effect, and 100 = complete control. A dash (-) response means no test.

The results are summarized in Table 2.

## Table 2

| | Compound I | | |
|---|---|---|---|
| Rate (g/ha) | 4 | 16 | 62 |
| POSTEMERGENCE | | | |
| Nutsedge | 0 | 0 | 0 |
| Crabgrass | 0 | 20 | 50 |
| Cassia | 40 | 80 | 90 |
| Teaweed | 20 | 50 | 80 |
| Rape | 80 | 100 | 100 |
| Jimsonweed | 60 | 90 | 90 |
| Velvetleaf | 80 | 90 | 100 |
| Blackgrass | 30 | 80 | 100 |
| Rice | 50 | 60 | 100 |
| Sugar Beets | 0 | 20 | 20 |
| Wheat | 0 | 60 | 80 |
| Wild Oats | 0 | 20 | 80 |
| Cocklebur | 0 | 40 | 80 |
| Morningglory | 40 | 80 | 90 |
| Cotton | 30 | 90 | 100 |
| Johnsongrass | 70 | 80 | 100 |
| Barnyardgrass | 0 | 30 | 40 |
| Giant Foxtail | 40 | 80 | 80 |
| Soybeans | 80 | 100 | 100 |
| Corn | 80 | 100 | 100 |

Table 2 (Cont'd)

| | Compound I | |
|---|---|---|
| Rate (g/ha) | 62 | 250 |
| PREEMERGENCE | | |
| Nutsedge | 0 | 0 |
| Crabgrass | 30 | 80 |
| Cassia | 0 | 50 |
| Teaweed | 0 | 60 |
| Rape | 20 | 70 |
| Jimsonweed | 0 | 60 |
| Velvetleaf | 60 | 80 |
| Blackgrass | 70 | 80 |
| Rice | 50 | 90 |
| Sugar Beets | 0 | 0 |
| Wheat | 0 | 20 |
| Wild Oats | 60 | 80 |
| Cocklebur | 0 | 0 |
| Morningglory | 0 | 40 |
| Cotton | 0 | 20 |
| Johnsongrass | 80 | 90 |
| Barnyardgrass | 30 | 80 |
| Giant Foxtail | 70 | 90 |
| Soybeans | 20 | 80 |
| Corn | 0 | 40 |

Test B

Seeds of the following crops and weeds are sown into 15 cm diameter pots containing Sassafras sandy loam soil: wheat (Tritium aestivum), barley (Hordeum vulgare), sugar beet Beta vulgaris), black nightshade ( Solanum nigrum), chickweed (Stellaria media), common lambsquarters Chenopodium album), Galium (Galium aparine), knotweed (Polygonum aviculare), Kochia (Kochia scoparia), Matricaria (Matricaria inodora), redroot pigweed (Amaranthus retroflexus), smartweed - (Polygonum persicaria ), speedwell (Veronica persica), wild buckwheat (Polygonum convolvulus), wild mustard (Brassica kaber), wild radish - (Raphanus raphanistrum), annual bluegrass (Poa annua), annual ryegrass (Lolium multiflorum), blackgrass (Alopecurus myosuroides ), green foxtail - (Setaria viridis), and wild oats (Avena fatua). Compound I was formulated in a non-phytotoxic solvent and applied to the plants as a foliar spray. Plants are treated at three stages: 1) preemergence, 2) postemergence when sugar beets are at the 1st

true leaf stage, and 3) postemergence when sugar beets have three leaves. Plants are grown in a temperature-controlled greenhouse for the duration of the experiment.

Weed control and crop injury are evaluated visually (3 to 4 weeks following compound application), using a scale of 0 to 100%, where 0 = no injury or control and 100 = complete death of the plants. The ratings represent averages of two tests run under identical conditions and are summarized in Table 3.

Compound I

| Leaf Stage | 1st | 3rd | 1st | 3rd | 1st | 3rd | 1st | 3rd |
|---|---|---|---|---|---|---|---|---|
| Rate (g/ha) | 16 | 16 | 30 | 30 | 63 | 63 | 125 | 125 |
| POSTEMERGENCE | | | | | | | | |
| Wheat | 42 | 48 | 62 | 48 | 62 | 68 | 92 | 100 |
| Barley | 58 | 90 | 95 | 95 | 100 | 98 | 98 | 100 |
| Sugar Beets | 0 | 0 | 0 | 0 | 0 | 0 | 12 | 12 |
| Black Nightshade | 0 | 0 | 12 | 0 | 85 | 0 | 15 | 25 |
| Chickweed | 45 | 55 | 98 | 95 | 100 | 98 | 100 | 100 |
| Lambsquarters | 68 | 70 | 98 | 95 | 100 | 85 | 100 | 78 |
| Galium | 38 | 20 | 98 | 92 | 92 | 90 | 100 | 88 |
| Knotweed | 25 | 20 | 62 | 0 | 62 | 0 | 60 | 25 |
| Kochia | 0 | - | 0 | - | 12 | - | 95 | - |
| Matricaria | 0 | 0 | 0 | 0 | 30 | 30 | 32 | 20 |
| Pigweed | 95 | 95 | 100 | 100 | 98 | 98 | 98 | 100 |
| Smartweed | 28 | 45 | 62 | 60 | 68 | 62 | 80 | 75 |
| Speedwell | 42 | 0 | 10 | 15 | 52 | 20 | 42 | 12 |
| Buckwheat | 0 | 48 | 0 | 0 | 100 | 0 | 60 | 20 |
| Mustard | 98 | 90 | 98 | 95 | 100 | 98 | 100 | 98 |
| Wild Radish | 92 | 95 | 95 | 100 | 100 | 98 | 100 | 98 |
| Bluegrass | 0 | 10 | 62 | 35 | 85 | 72 | 75 | 85 |
| Ryegrass | 0 | 0 | 0 | 10 | 10 | 20 | 35 | 50 |
| Blackgrass | 18 | 12 | 12 | 45 | 80 | 85 | 92 | 98 |
| Green Foxtail | 22 | 45 | 58 | 88 | 90 | 88 | 98 | 98 |
| Wild Oats | 18 | 68 | 95 | 88 | 100 | 100 | 100 | 98 |

Table 3 (Cont'd)

Compound I

| Rate (g/ha) | 16 | 30 | 63 | 125 |
|---|---|---|---|---|
| PREEMERGENCE | | | | |
| Wheat | 0 | 0 | 22 | 40 |
| Barley | 0 | 0 | 35 | 55 |
| Sugar Beets | 0 | 0 | 0 | 0 |
| Black Nightshade | 32 | 20 | 50 | 70 |
| Chickweed | 0 | 0 | 50 | 70 |
| Lambsquarters | 38 | 62 | 70 | 88 |
| Galium | 0 | 50 | 85 | 98 |
| Knotweed | 0 | 0 | 0 | 15 |
| Kochia | 0 | 95 | 20 | 0 |
| Matricaria | 25 | 55 | 90 | 95 |
| Pigweed | 12 | 10 | 60 | 98 |
| Smartweed | 20 | 28 | 45 | 62 |
| Speedwell | 0 | 45 | 50 | 35 |
| Buckwheat | 0 | 45 | 90 | 38 |
| Mustard | 20 | 85 | 98 | 98 |
| Wild Radish | 62 | 72 | 98 | 98 |
| Bluegrass | 0 | 12 | 38 | 55 |
| Ryegrass | 0 | 0 | 10 | 0 |
| Blackgrass | 0 | 20 | 42 | 65 |
| Green Foxtail | 0 | 0 | 48 | 62 |
| Wild Oats | 0 | 10 | 60 | 78 |

## Claims

1. 2-Butyl-N-[(4,6-dimethoxypyrimidin-2-yl)-aminocarbonyl]-2,3-dihydro-5-methoxy-1,2-benzisothiazole-7-sulfonamide, S,S-dioxide, and agriculturally suitable salts thereof.

2. A composition suitable for controlling the growth of undesired vegetation which comprises an effective amount of the compound of Claim 1 and at least one of the following: surfactant, solid or liquid diluent.

3. A method for controlling the growth of undesired vegetation which comprises applying to the locus to be protected an effective amount of a compound of Claim 1.

4. A method of Claim 3 wherein the locus to be

protected is a sugar beet crop.

5. A process for the preparation of a compound of claim 1, which comprises

(a) reacting an isocyanate of formula

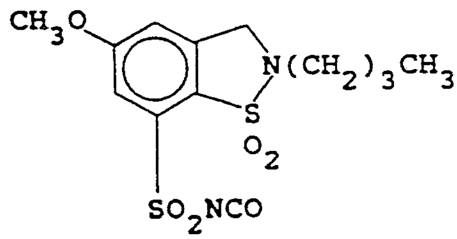

II

with an aminopyrimidine of formula

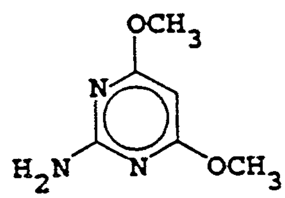

III

(b) reacting a sulfonamide of formula

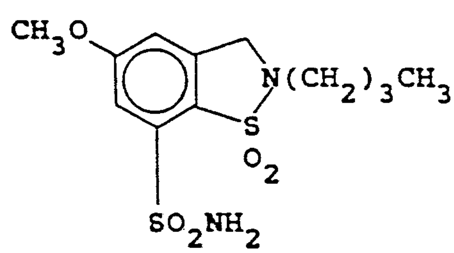

IV

with a phenyl carbamate of formula

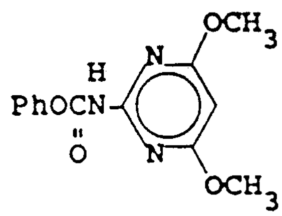

; or

V

(c) reacting the aminopyrimidine of formula (III) above with a phenylcarbamate of formula

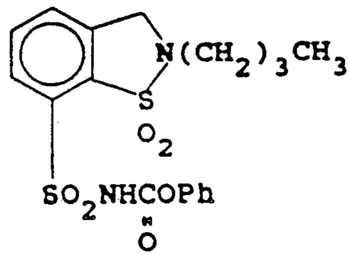

VI

Claims for the contracting state: AT

1. A process for the preparation of 2-butyl-N-[(4,6-dimethoxypyrimidin-2-yl)-aminocarbonyl]-2,3-dihydro-5-methoxy-1,2-benzisothiazole-7-sulfonamide, S.S-dioxide, and agriculturally suitable salts thereof, which comprises

(a) reacting an isocyanate of formula

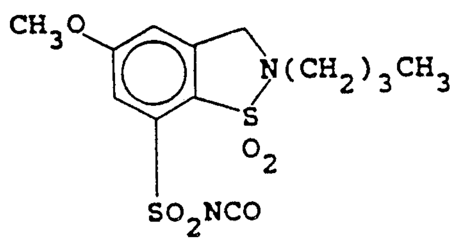

II

with an aminopyrimidine of formula

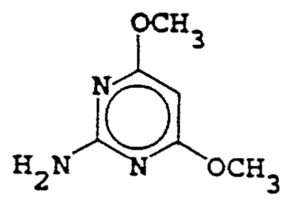

III

(b) reacting a sulfonamide of formula

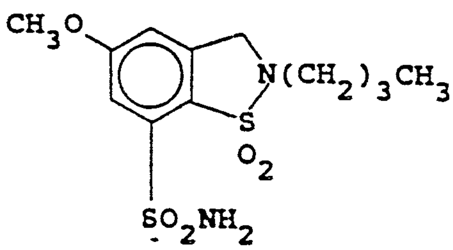

IV

with a phenyl carbamate of formula

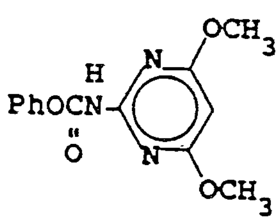

; or

V

(c) reacting the aminopyrimidine of formula (III) above with a phenylcarbamate of formula

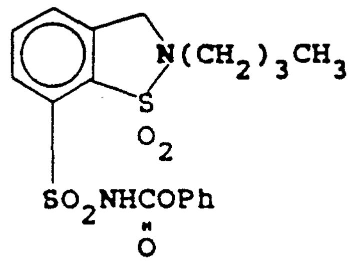

VI

2. A composition suitable for controlling the growth of undesired vegetation which comprises an effective amount of 2-butyl-N-[(4,6-dimethoxypyrimidin-2-yl)-aminocarbonyl]-2,3-dihydro-5-methoxy-1,2-benzisothiazole-7-sulfonamide, S,S-dioxide, and or an agriculturally suitable salt thereof and at least one of the following: surfactant, solid or liquid diluent.

3. A method for controlling the growth of undesired vegetation which comprises applying to the locus to be protected an effective amount of 2-butyl-N-[(4,6-dimethoxypyrimidin-2-yl)-aminocarbonyl]-2,3-dihydro-5-methoxy-1,2-benzisothiazole-7-sulfonamide, S,S-dioxide, or an agriculturally suitable salt thereof.

4. A method of Claim 3 wherein the locus to be protected is a sugar beet crop.

European Patent Office

# EUROPEAN SEARCH REPORT

Application number

EP 86 30 2805

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | EP-A-0 107 979 (E.I. DU PONT DE NEMOURS AND CO.) * claims 29, 30; page 13, lines 6-22; page 14, line 4 - page 15, line 3; page 61, example 8; page 64, table 1a, line 21 * --- | 1-3,5 | C 07 D 417/12<br>A 01 N 47/36 |
| P,A | EP-A-0 161 905 (E.I. DU PONT DE NEMOURS AND CO.) * claims 20, 21; page 13, lines 2-26; page 16, line 1 - page 17, line 6; page 24, General Structure 10; page 44, table 10, line 31 * --- | 1-3,5 | |
| P,A | EP-A-0 164 269 (E.I. DU PONT DE NEMOURS AND CO.) * claims 8, 9; page 4, formula I; page 7, line 12 - page 8, line 27; pages 61-64 * --- | 2,3,5 | |
| A | EP-A-0 125 864 (E.I. DU PONT DE NEMOURS AND CO.) * claims 12, 13; page 2, formula I; page 3, formula J-4; page 5, lines 24-35; page 19, table II-c * --- | 2,3,5 | TECHNICAL FIELDS SEARCHED (Int. Cl.4)<br>A 01 N 47/36<br>C 07 D 417/12<br>C 07 D 521/00 |
| A | EP-A-0 099 339 (CIBA-GEIGY) * claims 1-4, 26, 29 * & ZA - A - 83 5165 (Cat. D,A) ----- | 2,5 | |

The present search report has been drawn up for all claims

| Place of search | Date of completion of the search | Examiner |
|---|---|---|
| BERLIN | 11-08-1986 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document